# FASCICULE DE BREVET EUROPEEN

(11) **EP 3 074 313 B1**
(45) Date de publication et mention de la délivrance du brevet: **26.04.2017**
(21) Numéro de dépôt: 15720105.4
(22) Date de dépôt: 11.05.2015
(51) Int. Cl.: B65B 31/02, B65B 55/02, B65B 3/16, B65B 55/18, B65B 3/00, B65B 7/14, A61L 2/07, A61L 2/20, B65B 39/00

(54) **DISPOSITIF ET PROCÉDÉ DE REMPLISSAGE ASEPTIQUE**
VORRICHTUNG UND VERFAHREN ZUR ASEPTISCHEN FÜLLUNG
DEVICE AND METHOD FOR ASEPTIC FILLING

(30) Priorité: 09.05.2014 FR 1454174
(43) Date de publication de la demande: 05.10.2016
(73) Titulaire: Pierre Fabre Dermo-Cosmétique, 92100 Boulogne-Billancourt (FR)
(72) Inventeur: CORMARY, Bertrand, 81000 Albi (FR); SERRA, Laurent, 34260 La Tour sur Orb (FR)
(74) Mandataire: Ipside
(86) Numéro de dépôt international: PCT/EP2015/060394
(87) Numéro de publication internationale: WO 2015/169972

(56) Documents cités:
- EP-A1- 1 561 686
- EP-A1- 2 179 925
- EP-A2- 0 096 336
- WO-A1-96/29556
- WO-A1-98/19957
- WO-A1-2012/171958
- WO-A1-2013/007755
- WO-A2-2004/026735
- US-A1- 2008 184 671

## Description

L'invention concerne un dispositif et un procédé de remplissage aseptique. L'invention est plus particulièrement, mais non exclusivement, adaptée au conditionnement de produits dermo-cosmétiques ou galéniques, plus particulièrement au conditionnement de produits cosmétiques stériles.

Plus précisément le dispositif et le procédé objets de l'invention s'appliquent au conditionnement industriel automatisé, d'un produit stérile en vrac dans un contenant individuel stérile tel qu'une ampoule, un flacon, une seringue ou un tube, sans que cette liste ne soit exhaustive. Le produit conditionné se présente sous la forme d'un liquide, d'un gel ou d'une crème dont la viscosité est généralement comprise entre 600 Cps et 45 000 Cps (6 à 45 Pa.s). Le terme conditionnement stérile désigne ainsi le remplissage d'un contenant stérile par un produit stérile, le produit ainsi conditionné répondant à un degré de stérilité défini sans qu'aucune stérilisation postconditionnement ne soit réalisée.

Selon un exemple de contexte de mise en oeuvre de l'invention, celle-ci est utilisée pour le conditionnement d'un produit dermo-cosmétique stérile produit en grande quantité par un dispositif et un procédé tels que décrits dans le document WO 2013 007755.

Selon l'art antérieur, les produits dermo-cosmétiques stériles sans formulation biostatique, sont conditionnés dans des contenants de faible contenance afin de limiter les risques de rétro-contamination du produit après que le contenant ait été ouvert, ainsi, une fois le contenant ouvert, le produit doit être utilisé très rapidement. Ce conditionnement en petites quantités, unidose, et le faible temps de conservation après ouverture, limitent les possibilités de développement commercial de ces produits. Ces conditionnements unidose en milieu stérile sont généralement réalisés selon un procédé dit « *blow fill seal* » (soufflage, remplissage, scellage) qui ne permet pas d'intégrer un dispositif d'occlusion sophistiqué du contenant et reste donc limité à un conditionnement unidose.

Ces procédés de production de l'art antérieur, présentent l'inconvénient de coupler la production de contenants et la production de produit stérile. Par exemple, un stockage de produit implique le même stockage de contenant et vice-versa. Les contenants et le produit doivent être produits au même rythme, ce qui devient complexe à gérer en termes de flux de production lorsque le produit est présenté à la vente selon plusieurs conditionnements possibles.

L'utilisation d'un système de fermeture hermétique du contenant, tel que décrit dans le document FR 2 873 358 permet de supprimer les risques de rétro-contamination du produit et ouvre la voie vers des conditionnements plus habituels, par exemple en tube de plusieurs centaines de millilitres. Or, si des installations de conditionnement adaptées à des préparations non stériles existent pour ce type de contenant, il n'existe pas de chaîne de conditionnement stérile dans le domaine cosmétique ou galénique, apte à absorber un flux de production compatible avec un procédé de stérilisation tel que décrit dans le document WO 2013 007755.

Selon des modes de réalisation alternatifs de l'art antérieur, le produit est stérilisé après son conditionnement, notamment en soumettant ledit contenant comprenant le produit à un rayonnement ionisant. Ce mode de réalisation limite le choix du type de contenant, notamment de la matière le constituant, de sa forme, qui conditionne l'épaisseur du produit soumis à l'action ionisante, des encres utilisées pour la décoration du contenant, sans que cette liste ne soit exhaustive.

Selon l'art antérieur, appliqué au conditionnement non stérile, la préparation en vrac est placée dans une trémie et un dispositif de transfert amène le contenant sous ladite trémie pour son remplissage. Dans tout le texte, le terme « trémie » désigne un récipient comprenant une partie, dite de déversement, en forme de cône ou de pyramide renversée faisant entonnoir et dirigeant le contenant du récipient vers un orifice de déversement, ledit récipient étant cylindrique, de section circulaire, rectangulaire ou de tout autre forme

Selon l'art antérieur appliqué au conditionnement de produits alimentaires stérilisés, afin de protéger de toute contamination le vrac sortant de la trémie et remplissant le contenant, un flux d'air ultra-filtré est soufflé autour de la zone de remplissage. Ce flux d'air empêche les particules d'atteindre le produit déversé dans le contenant.

Le document WO 2012 171958 décrit une installation de conditionnement stérile pour denrées alimentaires. Dans cette installation, la zone de remplissage, éloignée de la trémie, se trouve dans une enceinte stérile protégée par un flux d'air laminaire du plafond vers le sol. Ces installations industrielles de l'art antérieur, adaptées à la production de gros volumes et conçues essentiellement pour les denrées alimentaires, et qui traitent en permanence la même matière, ne permettent pas d'obtenir les performances requises en matière de protection du vrac lors du remplissage, lorsqu'elles sont utilisées pour une application dans le domaine galénique ou dermo-cosmétique. En effet, dans le domaine de la galénique ou de la cosmétique stérile, les exigences de stérilité sont beaucoup plus élevées que dans l'alimentaire. Ainsi, selon la norme EN 556 relative aux dispositifs médicaux ainsi qu'à la pharmacopée européenne, un produit est dit stérile, lorsque son processus de stérilisation permet d'atteindre une valeur F0 de 15 minutes, c'est-à-dire l'équivalent d'une exposition à une température de 121 °C pendant 15 minutes. Pour les préparations cosmétiques stériles sans conservateur, le taux de stérilité à atteindre correspond à une probabilité pour la prolifération d'un micro-organisme de poliférer dans le produit, inférieure à 10⁻⁶. Ce niveau de stérilisation vis-à-vis de géobacillus stéarothermophilus sporulé est obtenu par un procédé de stérilisation thermique corrspondant à un F0 de 22 minutes. À titre comparatif, la stérilisation à ultra-haute température d'un produit alimentaire, tel que le lait correspond à un F0 de l'ordre 3 minutes.

Ce niveau de stérilité doit être conservé jusqu'au conditonnement final. Aussi, les turbulences générées dans le flux d'air protecteur dans la zone de remplissage du produit alimentaire selon le dispositif de l'artt antérieur, suffiraient à accroître de manière non admissible la probabilité de contamination du vrac dans le cas d'une application dans le domaine dermo-cosmétique. En effet, bien que l'environnement soit maintenu propre et stérile, compte tenu du volume d'une installation industrielle, il existe toujours un risque qu'une particule puisse être introduite dans le contenant ou dans la préparation au moment du remplissage. Paradoxalement, le flux d'air protecteur devient, du fait de ces turbulences, la principale cause de contamination dudit vrac. De plus, la production dans le domaine dermo-cosmétique procède par lot de produits dont les caractéristiques physiques, notamment la visosité, et les volumes individuel de conditionnement sont très variables. Aussi, la capacité de changer rapidement de lot de production sur la même chaîne de conditionnement est essentielle.

Dans les solutions de l'art antérieur mettant en oeuvre un conditionnement stérile, par exemple tel que l'enseigne le document EP 2 179 925, la trémie est éloignée de la zone de remplissage. Cette solution de l'art antérieur implique la présence d'une longueur de canalisation entre la trémie et la zone de remplissage. Or, toutes ces canalisations doivent être parfaitement nettoyées à chaque changement de lot, c'est-à-dire à chaque changement de type de produit conditionné. Ce nettoyage est particulièrement délicat, lorsque le produit conditionné est un produit demo-cosmétique visqueux sous la forme d'une crème ou d'un baume. De plus, toutes ces longueurs de canalisations comportent des zones dans lesquelles sont susceptibles de se développer des micro-organismes dans des traces de produit piégées.

L'invention vise à résoudre les inconvénients de l'art antérieur et concerne à cette fin un dispositif de conditionnement d'un vrac stérile, notamment une préparation dermo-cosmétique ou galénique, dans un contenant, lequel dispositif comprend :
a. une enceinte stérile et dans cette enceinte stérile,
b. une trémie, contenant le vrac, et comprenant selon une direction axiale une partie réservoir cylindrique et une partie de déversement raccordée à la partie cylindrique ;
c. un dispositif de transfert, apte à transporter sous la trémie le contenant en vue de son remplissage dans une zone dite de remplissage ;
d. un dispositif de soufflage d'air ultra-filtré du plafond vers le sol de l'enceinte, apte à produire un flux d'air, dit flux de protection, autour de la zone de remplissage ;
e. dans lequel l'axe de la partie de déversement de forme conique dissymétrique est excentré par rapport à l'axe de la partie réservoir de sorte à déporter l'orifice de déversement vers la paroi externe du réservoir.

Ainsi, bien que la trémie soit placée au plus proche de la zone de remplissage minimisant ainsi la longueur de canalisation entre le vrac et les contenants. La présence de cette trémie ne perturbe pas l'écoulement laminaire du flux d'air de protection dans la zone de remplissage, évitant ainsi les turbulences et éliminant les risques de contamination du produit au remplissage par des particules amenées par ledit flux d'air.

L'invention est avantageusement mise en oeuvre selon les modes de réalisation exposés ci-après lesquels sont à considérer individuellement ou selon toute combinaison techniquement opérante.

Avantageusement, le dispositif objet de l'invention comporte :
f. des moyens d'aspiration aptes à créer un flux d'air balayant le dispositif de transfert.

Ces moyens favorisent l'obtention d'un flux d'air protecteur laminaire autour de la zone de remplissage, et orientent toute particule susceptible de contaminer le produit vers les moyens d'aspiration ou elle est évacuée en dehors de la zone de remplissage.

Selon un mode de réalisation particulier, le dispositif objet de l'invention est adapté au remplissage d'un tube souple fermé à une extrémité, et comprend selon ce mode de réalisation :
g. des moyens pour maintenir le tube souple sur le dispositif de transfert et présentant l'extrémité ouverte dudit tube sous l'orifice de déversement ;
h. une station de scellage de l'extrémité ouverte du tube, desservie par le dispositif de transfert après la zone de remplissage ladite station de scellage étant sous le flux de protection.

Ainsi les tubes sont scellés en conditions stériles juste après leur remplissage et le produit déversé dans le tube ne reste exposé qu'un temps très réduit à l'air et toujours sous un flux de protection d'air ultra-filtré.

Avantageusement le tube est constitué d'un matériau thermoplastique et le sertissage est réalisé par une soudure bord à bord de l'extrémité ouverte du tube.

Ce mode de réalisation est particulièrement économique et rapide et assure une fermeture parfaitement étanche de l'extrémité ouverte du tube.

Avantageusement, l'extrémité initialement fermée du tube souple est un bouchon apte à éviter la rétro-contamination du produit contenu dans ledit tube. Ainsi le produit est avantageusement conditionné dans des contenants adaptés à une utilisation régulière.

Avantageusement, les moyens de maintien du tube souple sur le dispositif de transfert comportent un conteneur alvéolaire dans lequel sont insérés les tubes souples. Ainsi, chaque lot de tube est approvisionné stérile monté dans son conteneur, lequel est installé sur le dispositif de transfert du dispositif objet de l'invention pour le conditionnement du produit.

L'invention concerne également un procédé pour la fabrication et le conditionnement d'un vrac stérile, notamment une préparation dermo-cosmétique ou galénique, utilisant un dispositif de conditionnement selon l'invention et un dispositif de stérilisation par infusion comprenant un générateur de vapeur lequel procédé comporte les étapes consistant à :
i. stériliser le vrac dans un dispositif mettant en oeuvre un procédé par infusion de vapeur ;
ii. transférer le vrac ainsi stérilisé dans la trémie d'un dispositif de conditionnement selon l'invention ;
iii. approvisionner un ensemble stérile comprenant des contenants maintenus dans un conteneur alvéolaire ;
iv. conditionner le vrac dans les contenants selon un procédé de remplissage comprenant le passage desdits contenants dans leur conteneur alvéolaire sous la trémie dudit dispositif de conditionnement sous un flux laminaire ultra-filtré.

Ce procédé permet de réaliser des préparations dermo-cosmétiques ou galéniques stériles à partir de matières premières non stérilisées sans stérilisation post-conditionnement et ainsi offre une grande souplesse en termes de choix des matières premières, des conditionnements, tant en volume qu'en type de conditionnement (tube, ampoule) que de la matière constituant ledit conditionnement.

Selon une variante de ce procédé, l'étape ii) est réalisée par transfert direct du dispositif d'infusion vers la trémie. Cette variante de réalisation est adaptée à une production en flux tendu sur le même site géographique. La trémie fait office de volume tampon.

Selon une autre variante de réalisation, le procédé objet de l'invention comprend entre l'étape i) et l'étape ii) une étape consistant à :
v. stocker le vrac stérilisé dans une cuve stérile fermée, l'étape ii) étant réalisée par transfert du vrac de ladite cuve dans la trémie.

Cette variante de réalisation permet la stérilisation du vrac sur un site géographique et son conditionnement sur un autre. Plus généralement elle offre plus de souplesse dans la gestion de la production.

Selon un mode de réalisation particulièrement avantageux, le procédé objet de l'invention avant l'étape i) une étape consistant à :
vi stériliser l'ensemble de l'installation par la circulation dans ladite installation de vapeur à haute température produite par le moyen de stérilisation par infusion.

Ainsi, la vapeur produite par le dispositif utilisé pour la production, permet de stériliser l'ensemble de l'installation sans l'ajout de dispositif particulier. L'installation est ainsi adaptée à un changement rapide de série.

Le procédé objet de l'invention est avantageusement mis en oeuvre avec contenant sous la forme d'un tube d'une contenance comprise 50 ml et 400 ml comprenant un bouchon anti-rétrocontamination.

Selon ce mode de réalisation, le procédé objet de l'invention comprend avantageusement une étape consistant à :
viii. fermer le tube par une ligne de soudure après son remplissage. Ainsi le tube est hermétiquement fermé avant de quitter la zone stérile de conditionnement.

Avantageusement, le vrac contenu dans le tube après l'étape viii) est d'une stérilité telle que la probabilité de développement d'un micro-organisme dans la composition est inférieure à 10⁻⁶. Ainsi, le produit contenu dans le tube est apte à se conserver.

L'invention est exposée ci-après selon ses mode de réalisation préférés, nullement limitatifs, et en référence aux figures 1 à 5 dans lesquelles :
- la figure 1, relative à l'art antérieur, représente schématiquement un exemple de réalisation d'un dispositif de conditionnement stérile adapté, par exemple au conditionnement d'une denrée alimentaire ;
- la figure 2 montre selon une représentation schématique un exemple de réalisation d'un dispositif de conditionnement d'un vrac stérile selon l'invention ;
- la figure 3 illustre schématiquement un exemple de réalisation d'une installation de production et de conditionnement d'un produit derm o-cométique stérile ;
- la figure 4, montre un exemple de réalisation d'un contenant d'une préparation dermo-cosmétique stérile selon l'invention, figure 4A, selon une vue de face en coupe, avant le remplissage dudit contenant, maintenu dans un conteneur alvéolaire et figure 4B selon une vue de face après remplissage et sertissage ;
- et la figure 5 est un logigramme du procédé de conditionnement d'un vrac stérile selon l'invention.

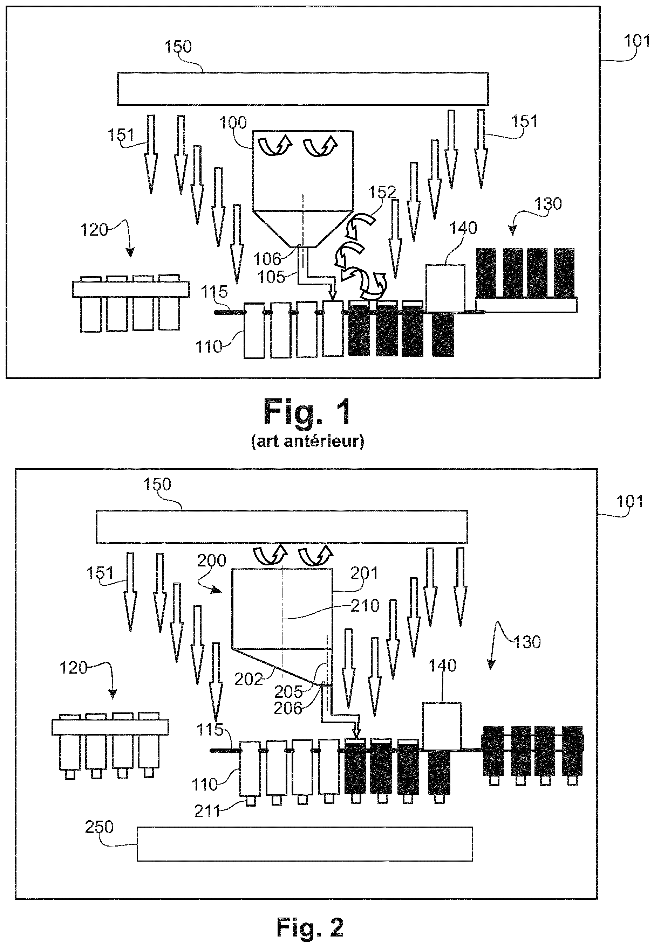

Figure 1, selon un exemple de réalisation de l'art antérieur appliqué notamment au cas du remplissage d'un contenant (110) par une denrée alimentaire, le vrac est contenu dans une trémie (100). Le produit est déversé de la trémie (100) dans le contenant (110) par un bec de remplissage connecté à l'orifice (106) de déversement de la trémie (100). Les contenants (110) sont portés par un dispositif (115) de transfert qui les amène d'un poste de chargement (120) à un poste de déchargement (130). L'ensemble de la machine est compris dans une enceinte (101) dont l'accès est réalisé par un sas. Après son remplissage le contenant (110) est fermé et scellé par un dispositif (140) d'obturation, avant d'être dirigé vers le poste (130) de déchargement. Pour assurer l'absence de contamination par des particules lors du remplissage, un dispositif de soufflage (150) génère un flux d'air ultra-filtré du plafond vers le sol du dispositif. Ce flux d'air (151) est majoritairement laminaire dans le volume de l'enceinte, cependant, la présence de la trémie (100) crée des turbulences (152) dans ce flux d'air, plus particulièrement au niveau de la zone de remplissage. Ces turbulences sont susceptibles de conduire à la contamination des contenants par des particules. Dans le cas où le vrac est un produit alimentaire, le risque de contamination engendré par ces turbulences est acceptable l'enceinte de la machine étant elle-même propre et stérilisée. Dans le cas d'un produit cosmétique sans conservateur, le taux de stérilité exigé ne permet pas de tolérer un tel risque de contamination.

Figure 2, afin de pallier ces inconvénients de l'art antérieur, le dispositif objet de l'invention utilise une trémie (200) spécifique, comprenant une partie (201) dite réservoir, cylindrique, suivie axialement d'une partie (202), dite de déversement, raccordée à la partie (201) cylindrique de forme conique dissymétrique. L'axe (205) sur lequel se trouve le sommet du cône définissant la forme de la partie (202) de déversement est excentré par rapport à l'axe (210) de la partie (201) cylindrique. Ainsi, l'orifice (206) de déversement de la trémie, orifice sensiblement centré sur l'axe (205) portant le sommet du cône de la forme de la partie (202) de déversement, est excentré vers la paroi externe de la partie (201) réservoir de ladite trémie (200). Cette disposition permet de conserver un flux laminaire dans la zone de remplissage du contenant et ainsi de limiter considérablement les risques de contamination du produit par des particules lors du remplissage. En effet, selon cette disposition, la partie (201) cylindrique, dite réservoir, ne masque pas la zone de remplissage vis-à-vis du flux (150) d'air provenant du plafond. Le dispositif objet de l'invention comprend, selon cet exemple de réalisation, un dispositif (250) d'aspiration est placé sous le dispositif (115) de transfert. Ce dispositif aspiration crée un balayage dudit dispositif de transfert par un flux d'air et favorise également, dans la configuration du dispositif objet de l'invention, la création d'un flux laminaire autour de la zone de remplissage. Selon cet exemple de réalisation, le contenant (110) est un tube dont une extrémité est ouverte pour le remplissage et l'autre extrémité est fermée par un bouchon (211) spécifique, apte à éviter toute rétro-contamination du produit contenu dans le tube lors de l'utilisation dudit tube par le consommateur. L'enveloppe du tube (110) est constituée d'un matériau thermoplastique et la fermeture du contenant est réalisée par soudure des bords de l'extrémité ouverte. Selon cet exemple de réalisation lesdits tubes ont une contenance comprise en 50 ml et 450 ml sans que ces valeurs ne soient limitatives. Selon cet exemple, le vrac est un produit dermo-cosmétique se présentant sous la forme d'une émulsion dont la viscosité est comprise entre 600 Cps et 45 000 Cps.

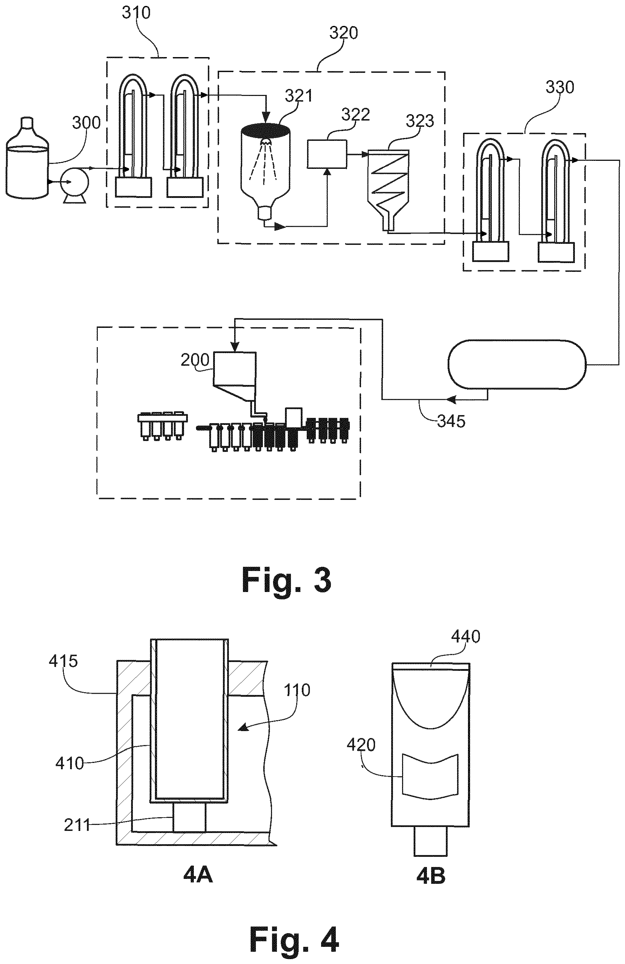

Figure 3, selon un exemple de réalisation du procédé objet de l'invention, la préparation (300) dermo-cosmétique ou galénique est stérilisée par la chaleur selon un procédé dit UHT pour « ultra-haute température ». À cette fin, et selon cet exemple de réalisation, au cours d'une étape (310) de préchauffage, ladite préparation est amenée à une température adéquate, notamment pour lui conférer une viscosité adaptée au traitement de stérilisation visée. Selon cet exemple de réalisation, l'étape (320) de stérilisation est réalisée par infusion. Selon ce procédé, la préparation préchauffée est introduite sous forme d'une douche dans une enceinte (321) comprenant de la vapeur d'eau propre à une température définie. Le chauffage de la préparation est instantané du fait de la surface d'échange importante entre les filets de la préparation et la vapeur, et de la condensation de la vapeur sur lesdits filets. La préparation est maintenue (322) à cette température durant un temps très court, fonction du niveau de stérilisation visé, avant d'être injectée dans une chambre (323) sous vide où ladite préparation subit un refroidissement très rapide, du fait de la vaporisation, sous l'effet du vide, de l'eau condensée au cours de la phase de chauffage (321). La préparation est ensuite refroidie (330) progressivement sous agitation jusqu'à une température favorable pour son conditionnement, par exemple une température comprise entre 30 °C et 40 °C. La préparation est, selon cet exemple de réalisation, stockée dans une cuve tampon fermée. Ladite cuve tampon est, selon un exemple de réalisation du procédé objet de l'invention, connectée par une canalisation (345) à la trémie (200) du dispositif de conditionnement objet de l'invention. Les conditions de stérilisation, à savoir la température et la vitesse de préchauffage, la température et la durée de la stérilisation par infusion ainsi que la vitesse de refroidissement après la stérilisation sont sélectionnées de sorte que l'émulsion constituant la préparation ne soit pas cassée au cours de ce processus, c'est-à-dire que ladite préparation ne perde pas son caractère émulsif et qu'elle conserve ses caractéristiques organoleptiques. Ainsi, la préparation stérilisée par le procédé d'infusion est transférée sous forme de vrac stérile dans le dispositif de conditionnement sans autre traitement. Le transfert du vrac dans la trémie (200) est réalisé par des canalisations (345) stériles de sorte que ledit vrac n'est pas en contact avec l'air, ce qui élimine tout risque de contamination avant le remplissage. Avantageusement, selon cette configuration de mise en oeuvre du procédé objet de l'invention, le générateur de vapeur du dispositif de stérilisation par infusion (320) est utilisé pour stériliser l'ensemble de l'installation, par la circulation de vapeur dans l'ensemble du circuit de production - conditionnement, c'est-à-dire notamment les canalisations, les dispositifs de chauffage - refroidissement, la cuve tampon et la trémie (200), lorsque ledit circuit est vidé de la présence de tout produit. Alternativement, la préparation stérile est stockée dans la cuve tampon étanche, et transportée sur un lieu de conditionnement distant du lieu de stérilisation.

Figure 4A, préalablement à l'opération de conditionnement, les tubes (110) stériles vides, sont installés dans un conteneur (415) alvéolaire, par exemple constitué de plaques de polypropylène alvéolaires. Les bouchons (211) spécifiques, empêchant la rétro-contamination, sont installés à l'extrémité desdits tubes (110). L'ensemble est stérilisé et ensaché. À titre d'exemple non limitatif, l'enveloppe (410) du tube (110) est constituée de polyéthylène monocouche. Selon un mode de réalisation alternatif, l'enveloppe du tube est multicouche et comprend une couche d'un polymère imperméable à l'oxygène. Selon des modes de réalisation alternatifs, l'enveloppe (410) du tube (110) est constituée d'un matériau métallique. En pratique, le procédé et le disopositif objet de l'invention n'impliquant pas de stérilisation post-conditionnement, le choix de matière constituant le tube est très large.

Figure 4B, les tubes remplis ne subissant pas d'autre opération de stérilisation, les tubes vides sont installés imprimés dans les conteneurs alvéolaires. Avantageusement le tube (110) et ledit conteneur (415) comprennent des repères (non représentés) aptes à coopérer pour orienter correctement la partie (420) imprimée du tube en regard de la ligne (440) de soudure réalisant la fermeture du tube une fois celui-ci rempli.

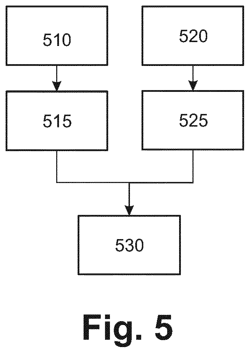

Figure 5, le procédé objet de l'invention permet d'obtenir selon un mode de production industriel, avec des flux de production de l'ordre de 2000 Kg par heure, un produit dermo-cosmétique ou galénique stérile, sans conservateur, dans un contenant directement proposable à la vente au détail et comprenant toutes les mentions obligatoires et les signes distinctifs appropriés. Ledit procédé comprend une étape (510) de production d'un vrac stérile, notamment par un procédé de stérilisation par infusion, et, en parallèle, une étape (520) d'approvisionnement de contenants stériles en conteneur, dit ensemble stérile, notamment dans un conteneur alvéolaire ensaché. Ainsi, contrairement aux procédés de l'art antérieur de type « *blow fill seal* » pour des conditionnements unidose, ou de stérilisation du produit dans le contenant, le procédé objet de l'invention permet de découpler les deux flux de production et ainsi d'offrir une flexibilité de production accrue tout en conservant les avantages du flux tendu. Selon une étape (525) de préparation des contenants, le conteneur est extrait de son ensachage et installé sur la table du dispositif de conditionnement selon l'invention, le tout en milieu propre. Parallèlement, selon une étape (515) d'approvisionnement du vrac, le vrac stérile produit à l'étape (510) précédente est introduit dans la trémie du dispositif de remplissage objet de l'invention. Selon des variantes du procédé objet de l'invention, le vrac ainsi introduit dans la trémie provient directement du dispositif de stérilisation ou, la trémie est alimentée depuis une cuve stérile. Les contenants et le vrac étant transférés dans le dispositif de conditionnement, les contenants sont remplis et scellés au cours d'une étape (530) de remplissage.

À titre d'exemple, le procédé et le dispositif objets de l'invention permettent de produire un produit stérile distribué en contenant tubulaire d'une contenance comprise en 50 ml et 400 ml. Ledit produit se trouve sous la forme d'une émulsion, la composition conditionnée dans le contenant étant stérile, avec une probabilité inférieure à 10⁻⁶ de prolifération d'un micro-organisme, tout en étant dépourvue de conservateur. Ce type de produit, destiné à être étalé sur la peau, ne peut-être présenté en conditionnement unidose.

Un produit « sans conservateur » désigne un produit exempt de composés visant à prévenir la prolifération de bactéries ou de champignons dans la formule, tels que, notamment, les produits désignés par paraoxybenzoates ou parabènes, le triclosan, le citromonium bromide, le méthylisothiazolinone, les formaldéhydes, le phénoxyéthanol ou les détergents sulfatés, sans que cette liste ne soit exhaustive.

Ainsi, l'utilisation de la combinaison du procédé de stérilisation et de conditionnement objet de l'invention permet de proposer un tel produit stérile, sans conservateur, dans un conditionnement qui permet une utilisation et un étalement facile. En effet, le tube, muni de son bouchon anti-rétrocontamination, permet de prélever une noisette de produit avant de l'étaler sur la peau, et de renouveler cette manipulation autant que nécessaire pour couvrir toute la surface d'application, sans gaspillage de produit et tout en conservant le produit stérile.

Selon un exemple de réalisation le procédé et le dispositif objet de l'invention sont mis en oeuvre pour la fabrication et le conditionnement d'une crème, stérile sans conservateur comprenant :
- 2 % à 5 % cetearyl glucoside ;
- 0,1 % à 0,5 % de carbomer ;
- 5 % à 12 % de glycerine ;
- 2 % à 10 % de triglycéride caprique caprylique ;
- 5 % à 10 % de paraffine liquide ;
- de l'eau en complément à 100%.

Selon cet exemple de mise en oeuvre, le produit est conditionné en tubes contenant 50 ml à 400 ml de produit et fermés par un bouchon anti-rétrocontamination. Ainsi le procédé et le dispositif objets de l'invention permettent de stériliser et de conditionner des produits qui, du fait de leur viscosité ou de la présence de composés solides ou cireux, ne peuvent être stérilisés par le passage au travers d'une membrane et dont les contenants visés se prêtent mal à la stérilisation dans le contenant.

La description ci-avant et les exemples de réalisation montrent que l'invention atteint les objectifs visés, en particulier elle permet selon un processus de production industriel flexible et en flux tendus, de produire une préparation dermo-cosmétique ou galénique stérile, selon des débits de production élevés en assurant le respect d'une stérilité élevée, c'est-à-dire avec un F0 supérieur à 15 minutes, tout le long de la chaîne de production. Elle permet de proposer des produits stériles sans conservateur dans des contenants de gros volumes, pemettant une application aisée et économique desdits produits.

## Revendications

1. Dispositif de conditionnement d'un vrac stérile, notamment une préparation dermo-cosmétique ou galénique, dans un contenant (110), comprenant :
a. une enceinte (101) stérile et dans cette enceinte stérile,
b. une trémie (200), contenant le vrac, et comprenant selon une direction axiale une partie (201) réservoir cylindrique et une partie (202) de déversement raccordée à la partie (201) cylindrique ;
c. un dispositif (115) de transfert, apte à transporter sous la trémie (200) le contenant (110) en vue de son remplissage dans une zone dite de remplissage ;
d. un dispositif (150) de soufflage d'air ultra-filtré du plafond vers le sol de l'enceinte (101), apte à produire un flux d'air, dit flux de protection, autour de la zone de remplissage ;
e. **caractérisé en ce que** l'axe (205) de la partie (202) de déversement de forme conique dissymétrique est excentré par rapport à l'axe (210) de la partie (201) réservoir de sorte à déporter l'orifice (206) de déversement vers la paroi externe du réservoir.

2. Dispositif selon la revendication 1, comportant :
f. des moyens (250) d'aspiration aptes à créer un flux d'air balayant le dispositif (115) de transfert.

3. Dispositif selon la revendication 1, dans lequel le contenant (110) est un tube souple, fermé à une extrémité, lequel dispositif comprend :
g. des moyens (415) pour maintenir le tube souple sur le dispositif de transfert et présentant l'extrémité ouverte dudit tube (110) sous l'orifice de déversement ;
h. une station de scellage (140) de l'extrémité ouverte du tube, desservie par le dispositif de transfert après la zone de remplissage, ladite station de scellage étant sous le flux de protection.

4. Dispositif selon la revendication 3, dans lequel le tube (110) est constitué d'un matériau thermoplastique et où le scellage est réalisé par une soudure (440) bord à bord de l'extrémité ouverte dudit tube.

5. Dispositif selon la revendication 3, dans lequel l'extrémité initialement fermée du tube souple est un bouchon (211) apte à éviter la retro-contamination du produit contenu dans ledit tube.

6. Dispositif selon la revendication 3, dans lequel les moyens de maintien du tube souple sur le dispositif de transfert comportent un conteneur (415) alvéolaire dans lequel sont insérés les tubes (110) souples.

7. Procédé pour la fabrication et le conditionnement d'un vrac stérile, notamment une préparation dermo-cosmétique ou galénique, utilisant un dispositif de conditionnement selon la revendication 1 et un dispositif (320) de stérilisation par infusion comprenant un générateur de vapeur, **caractérisé en ce qu'**il comporte les étapes consistant :
i. stériliser (510) le vrac dans ledit dispositif (320) de stérilisation par infusion mettant en oeuvre un procédé par infusion de vapeur ;
ii. transférer (515) le vrac ainsi stérilisé dans la trémie d'un dispositif de conditionnement selon la revendication 1 ;
iii. approvisionner (520) un ensemble stérile comprenant des contenants montés dans un conteneur alvéolaire ;
iv. conditionner (530) le vrac dans les contenants selon un procédé de remplissage comprenant le passage desdits contenants dans leur conteneur alvéolaire sous la trémie dudit dispositif de conditionnement sous un flux laminaire ultra-filtré.

8. Procédé selon la revendication 7, dans lequel l'étape ii) est réalisée par transfert direct depuis le dispositif d'infusion de vapeur vers la trémie.

9. Procédé selon la revendication 7, comprenant entre l'étape i) et l'étape ii) une étape consistant à :
v. stocker le vrac stérilisé dans une cuve stérile fermée, l'étape ii) étant réalisée par transfert du vrac de ladite cuve dans la trémie.

10. Procédé selon la revendication 7, comprenant avant l'étape i) une étape consistant à :
vi stériliser l'ensemble de l'installation comprenant ledit dispositif de conditionnement selon la revendication 1 et ledit dispositif (320) de stérilisation par infusion, par la circulation dans ladite installation de vapeur à haute température produite par le dispositif de stérilisation par infusion.

11. Procédé selon la revendication 7, dans lequel le contenant est un tube d'une contenance comprise 50 ml et 400 ml comprenant un bouchon anti-rétrocontamination

12. Procédé selon la revendication 11, comprenant une étape consistant à :
viii. fermer le tube par une ligne de soudure après son remplissage

13. Procédé selon la revendication 12, dans lequel le vrac comprend :
- 2 % à 5 % cetearyl glucoside ;
- 0,1 % à 0,5 % de carbomer ;
- 5 % à 12 % de glycerine ;
- 2 % à 10 % de triglyceride caprique caprylique ;
- 5 % à 10 % de paraffine liquide ;
- de l'eau en complément à 100%.
à l'exclusion de tout conservateur.

## Patentansprüche

1. Konditioniervorrichtung für ein steriles loses Gut, insbesondere für eine hautkosmetische oder galenische Präparation in einem Behältnis (110),
umfassend:
a. eine sterile Kammer (101) und in dieser sterilen Kammer
b. einen das lose Gut enthaltenden Trichter (200), der in einer axialen Richtung einen zylinderförmigen Behälterteil (201) und einen am zylinderförmigen Teil (201) angeschlossenen Auslaufabschnitt (202) umfasst;
c. eine Übertragungsvorrichtung (115), die das Behältnis (110) unter den Trichter (200) in Hinblick auf sein Einfüllen in einem sogenannten Einfüllbereich transportieren kann;
d. eine von der Decke bis zum Boden der Kammer (101) agierende Blaseinrichtung (150) für ultrafiltrierte Luft, die einen sogenannten Schutzluftstrom um den Einfüllbereich herum erzeugen kann,
**dadurch gekennzeichnet, dass** die Achse (205) des unsymmetrischen kegelförmigen Auslaufabschnitts (202) in Bezug zur Achse (210) des Kammerteils (201) derart außermittig ist, dass die Auslauföffnung (206) in Richtung der Außenwand des Behälters verschoben ist.

2. Vorrichtung nach Anspruch 1, umfassend:
f. Ansaugmittel (250), die einen über die Übertragungsvorrichtung (115) strömenden Luftstrom erzeugen.

3. Vorrichtung nach Anspruch 1, bei der das Behältnis (110) eine biegsame, an einem Ende geschlossene Tube ist, wobei die Vorrichtung umfasst:
g. Mittel (415), um die biegsame Tube auf der Übertragungsvorrichtung zu halten und das offene Ende der Tube (110) unter der Auslauföffnung zu präsentieren;
h. eine Abdichtstation (140) für das offene Tubenende, die von der Übertragungsvorrichtung nach dem Einfüllbereich gesteuert wird, wobei die Abdichtstation unter dem Schutzstrom liegt.

4. Vorrichtung nach Anspruch 3, bei der die Tube (110) aus einem thermoplastischen Material besteht und das Abdichten durch Stumpfschweißen (440) des offenen Tubenendes realisiert wird.

5. Vorrichtung nach Anspruch 3, bei der das ursprünglich geschlossene Ende der biegsamen Tube ein Verschluss (211) ist, der die Rückkontaminierung des in der Tube enthaltenen Produkts verhindern kann.

6. Vorrichtung nach Anspruch 3, bei der die Mittel zum Halten der biegsamen Tube auf der Übertragungsvorrichtung einen wabenförmigen Behälter (415) umfassen, in den die biegsamen Tuben (110) gesteckt werden.

7. Verfahren für die Herstellung und Konditionierung eines sterilen losen Guts, insbesondere einer hautkosmetischen oder galenischen Präparation, bei dem eine Konditioniervorrichtung nach Anspruch 1 und eine Aufgusssterilisationsvorrichtung (320) mit einem Dampfgenerator zum Einsatz kommen,
**dadurch gekennzeichnet, dass** es folgende Arbeitsschritte umfasst:
i. Sterilisieren (510) des losen Guts in der Aufgusssterilisationsvorrichtung (320), bei dem ein Verfahren durch Dampfaufguss zum Einsatz kommt;
ii. Übertragen (515) des so sterilisierten losen Guts in den Trichter einer Konditioniervorrichtung nach Anspruch 1;
iii. Auffüllen (520) einer sterilen Einheit mit in einen wabenförmigen Behälter montierten Behältnissen;
iv. Konditionieren (530) des losen Guts in die Behältnisse nach einem Auffüllverfahren, das das Vorbeifahren der Behältnisse in ihrem wabenförmigen Behälter unter dem Trichter der Konditioniervorrichtung unter einem ultrafiltrierten Laminar-Flow umfasst.

8. Verfahren nach Anspruch 7, bei dem der Arbeitsschritt ii) durch direkte Übertragung von der Dampfaufgussvorrichtung zum Trichter realisiert wird.

9. Verfahren nach Anspruch 7, das zwischen dem Arbeitsschritt i) und dem Arbeitsschritt ii) folgenden Arbeitsschritt umfasst:
v. Lagern des sterilisierten losen Guts in einem geschlossenen sterilen Kessel, wobei der Arbeitsschritt ii) durch Übertragen des losen Guts vom Kessel in den Trichter bewerkstelligt wird.

10. Verfahren nach Anspruch 7, das vor dem Arbeitsschritt i) folgenden Arbeitsschritt umfasst:
vi. Sterilisieren der kompletten Installation, die die Konditioniervorrichtung nach Anspruch 1 und die Aufgusssterilisationsvorrichtung (320) umfasst, durch Zirkulation in der Dampfanlage bei hoher Temperatur, die von der Aufgusssterilisationsvorrichtung erzeugt wird.

11. Verfahren nach Anspruch 7, bei dem das Behältnis eine Tube mit einem Füllvermögen zwischen 50 ml und 400 ml und einen eine Retrokontamination verhindernden Verschluss umfasst.

12. Verfahren nach Anspruch 11, das folgenden Arbeitsschritt umfasst: viii. Schließen der Tube nach dem Auffüllen durch eine Schweißnaht.

13. Verfahren nach Anspruch 12, bei dem das lose Gut umfasst:
- 2 % bis 5 % Cetearylglucosid;
- 0,1 % bis 0,5 % Carbomer;
- 5 % bis 12 % Glycerin;
- 2 % bis 10 % Capryl-Caprin-Triglycerid;
- 5 % bis 10 % flüssiges Paraffin;
- Wasser zum Ergänzen auf 100%
und keinerlei Konservierungsmittel.

## Claims

1. A device for packaging sterile bulk material, particularly a dermocosmetic or galenic preparation, into a container (110), comprising:
a. a sterile enclosure (101), and in that sterile enclosure,
b. a hopper (200) containing the bulk product and comprising in an axial direction a cylindrical reservoir part (201) and a pouring part (202) connected to the cylindrical part (201);
c. a transfer device (115) suitable for transporting the container (110) under the hopper (200) in order to fill the container in a zone known as the filling zone;
d. a device (150) for blowing ultra-filtered air from the ceiling to the floor of the enclosure (101), suitable for producing a flow of air known as a protective flow, around the filling zone;
e. **characterized in that** the axis (205) of the pouring part (202) with an asymmetrical conical shape is not in line with the axis (210) of the reservoir part (201) so that the pouring opening (206) is shifted towards the outer wall of the reservoir.

2. A device according to claim 1, comprising:
f. extraction means (250) suitable for creating an air flow that scavenges the transfer device (115).

3. A device according to claim 1, wherein the container (110) is a flexible tube, closed at one end, which device comprises:
g. means (415) for maintaining the flexible tube on the transfer device and
holding the open end of said tube (110) under the pouring opening;
h. a station (140) for sealing the open end of the tube, that is supplied by the transfer device after the filling zone, wherein said sealing station is under the protective flow.

4. A device according to claim 3, wherein the tube (110) is made of thermoplastic material and crimping is carried out by welding (440) together the edges of the open end of said tube.

5. A device according to claim 3, wherein the initially closed end of the flexible tube is a cap (211) suitable for avoiding retrograde contamination of the product contained in said tube.

6. A device according to claim 3, wherein the means for holding the flexible tube on the transfer device comprises a cellular collector (415) in which the flexible tubes (110) are inserted.

7. A method for manufacturing and packaging a sterile bulk product, particularly a dermocosmetic or galenic preparation, using a packaging device according to claim 1 and a device (320) for steam infusion sterilization comprising a steam generator, **characterized in that** it comprises the steps of:
i. sterilizing (510) the bulk product in a device that uses a steam infusion method;
ii. transferring (515) the bulk product thus sterilized to the hopper of a packaging device according to claim 1 ;
iii. supplying (520) a sterile assembly comprising containers held in a cellular collector;
iv. packaging (530) the bulk product in the containers using a filling method comprising passing said containers in their cellular collector under the hopper of said packaging device under an ultra-filtered laminar flow.

8. A method according to claim 7, wherein step (ii) is carried out by direct transfer from the steam infusion device to the hopper.

9. A method according to claim 7, comprising, between step (i) and step (ii), a step of:
v. storing the sterilized bulk material in a closed sterile tank (340), wherein step (ii) is carried out by transferring the bulk product from said tank into the hopper.

10. A method according to claim 7, comprising, prior to step (i), a step of:
vi sterilizing the whole system by circulating throughout said system high-temperature steam produced by the steam infusion device.

11. A method according to claim 7, wherein the container is a tube with a capacity ranging between 50ml and 400ml comprising a cap that prevents retrograde contamination.

12. A method according to claim 11, comprising a step of: viii. closing the tube with a weld line after it is filled.

13. A method according to claim 12, wherein the product comprises:
- 2% to 5% cetearyl glucoside;
- 0.1 % to 0.5% carbomer;
- 5% to 12% glycerin;
- 2% to 10% caprylic capric triglyceride;
- 5% to 10% liquid paraffin;
- water to make up 100%.
to the exclusion of any preservative.
